# EUROPEAN PATENT SPECIFICATION

(11) **EP 3 436 107 B1**
(45) Date of publication and mention of the grant of the patent: **07.06.2023**
(21) Application number: 16716120.7
(22) Date of filing: 30.03.2016
(51) Int. Cl.: A61M 5/14

(54) **ENTERAL FEEDING BAG AND PUMP SUPPORT**
BEUTEL ZUR ENTERALEN ERNÄHRUNG UND PUMPENHALTERUNG
POCHE D'ALIMENTATION ENTÉRALE ET SUPPORT DE POMPE

(43) Date of publication of application: 06.02.2019
(73) Proprietor: Covidien LP, Mansfield, MA 02048 (US)
(72) Inventor: HARRIS, Benjamin, Plainville, MA 02762 (US); WIESNER, Joel, O'Fallon, MO 63366 (US); MARKWARDT, Jeffrey, O'Fallon, Il 62269 (US); BREITWEISER, Kenneth, Brighton, IL 62012 (US); BIERMANN, Wayne, St. Charles, MO 63304 (US)
(74) Representative: Marks & Clerk LLP
(86) International application number: PCT/US2016/024900
(87) International publication number: WO 2017/171748

(56) References cited:
- WO-A1-2004/082560
- WO-A1-2015/010060
- US-A1- 2005 040 126
- US-A1- 2005 269 464

## Description

### TECHNICAL FIELD

This invention relates to a support system for a medical bag. More particularly, the invention relates to a support system for an enteral feeding bag that includes a medical device mounting assembly.

### BACKGROUND

Medical bags such as IV/enteral feeding bags are typically suspended from an IV pole, tabletop bag hangers, or straps within a backpack. Medical devices such as enteral feeding pumps are typically attached to a support structure such as an IV pole, a bed rail, or other support structure by means of a pole clamp or other attachment device that holds the pump in a fixed position relative to the support structure. During medical treatment, such as enteral feeding, it can be difficult to position the IV pole and medical bag in close proximity to the medical device. As a result, the configuration of the medical bag and medical device support structures of the prior art can cause use of the devices to be cumbersome, and may severely restrict the patient's mobility or be unsuitable for bedside use.

WO 2015/010060 A1 discloses mobility assistance devices for medical settings where a patient ambulation is desired but the patient remains connected to one or many medical components. The system comprises a mast connected to a base, wherein the mast is angled with respect to the base and from which any number of medical components is supported.

US 2005/040126 A1 discloses an apparatus for concealed transport of a medical fluid administration device. The medical fluid administration device is capable of infusing medical fluids to, or collecting medical fluids from, as the case may be, the body of a patient during concealed transport.

WO 2004/082560 A1 discloses a portable enteral feeding apparatus that facilitates ambulatory enteral feeding and prevents damage to an enteral feeding tube during use. The apparatus includes a backpack type enteral feeding support, a soft pouch having a hook and loop surface on the pouch and a rigid spacer.

US 2005/269464 A1 discloses a transportable intravenous stand for carrying medical fluids, such as blood plasma or saline solutions, so as to allow the fluids to be gravity fed into a patient comprising a base, a collapsible support member, and a hanger member to allow for hanging fluid-dispensing containers.

### SUMMARY

The present invention is defined in claim 1. Additional features of preferred embodiments thereof are defined in the dependent claims. i.e. the present invention can be directed to an IV or an enteral feeding bag support for use in a medical environment to suspend a medical bag above a support surface. The enteral feeding bag support of the present invention comprises a base assembly comprising a support portion for supporting the support on the support surface and an attachment portion for removably attaching a medical device to the support. A pole assembly comprises a medical bag attachment portion for attaching a medical bag to the pole assembly. The pole assembly is mountable on the base assembly such that when the pole assembly is mounted on the base and the base is on the surface, the medical bag attachment portion is disposed above the base suspending the medical bag above the support surface.

The system further comprises at least one foot movably attached to the support portion for movement between a deployed position and a stowed position wherein the at least one foot is releasably lockable in incremental positions between the stowed position and the deployed position. In the stowed position, each foot is received in a pocket in a bottom of the support portion such that each foot is substantially within an outer perimeter of the support portion. The foot may be pivotably attached to the support portion for pivoting between the deployed and stowed positions. The foot may be slidably attached to the support portion for translational movement between the deployed and stowed positions. The pole assembly can comprise a first elongate member and a second elongate member telescopingly received in the first elongate member for adjusting a relative position of the first and second elongate members. The second elongate member can comprise a first straight section for receipt in the first elongate member and a second curved section extending from the first straight section for attaching the medical bag. A lock may secure the second elongate member at a selected position relative to the first elongate member. The enteral feeding bag support further comprises a medical device mounting assembly. The attachment portion can mount the medical device mounting assembly to the base. The support portion and the attachment portion can be formed as one piece of material. The enteral feeding bag support may be combined with a backpack. The base of the support can have a first configuration for supporting the medical bag on the support surface and a second configuration for supporting the medical bag in the backpack.

The disclosure can be directed to an enteral feeding bag support for use in a medical environment to suspend a medical bag above a support surface. The enteral feeding bag support can comprise a base comprising a support portion for supporting the support on the support surface and an attachment portion. A medical device mounting assembly may be movably attached to the attachment portion of the base to attach a medical device to the base. A pole assembly can comprise a medical bag attachment portion for attaching a medical bag to the pole assembly. The pole assembly may be mountable on the base such that when the pole assembly is mounted on the base and the base is on the support surface, the medical bag attachment portion is disposed above the base suspending the medical bag above the support surface.

The support portion and the attachment portion may be formed as one piece of material. The support portion can comprise at least one foot attached to the support portion for engaging the support surface. The support portion can comprise feet attached to the support portion for engaging the support surface. The feet may be selectively movable with respect to the base between a stowed position and a deployed position. The feet may be attached to the support portion for incremental movement from the stowed position to the deployed position. The enteral feeding bag support can be combined with a backpack. The base of the support may have a first configuration for supporting the medical bag on the support surface and a second configuration for supporting the medical bag in the backpack. The base may have padding at a back of the base for engaging a wearer of the backpack when the support is in the backpack.

The present invention also relates to a combination backpack and medical fluid bag support system, comprising the medical fluid bag support system of the present invention in combination with a backpack, comprising a backpack portion configured to be supported on a wearer's shoulder and defining an internal storage compartment sized to receive therein the medical fluid bag support system.

### BRIEF DESCRIPTION OF THE DRAWINGS

FIG. 1 is a perspective view of a enteral feeding bag support with feet shown in a deployed position;
FIG. 2 is a top view of the support;
FIG. 3 is a bottom view of the support;
FIG. 4 is an enlarged fragmentary view of the support shown in FIG. 3;
FIG. 5 is a front view of the support showing a pole assembly in a first position;
FIG. 6 is a front view of the support showing the pole assembly in a second, extended position;
FIG. 7 is a perspective of the support with feet shown in a stowed position;
FIG. 8 is a bottom view of the support of Fig. 7;
FIG. 9 is an exploded view of the support;
FIG. 10 is a front and right side perspective of a base of the support;
FIG. 11 is a front and left side perspective of the base of the support;
FIG. 12 is a perspective of a medical device mounting assembly of the support;
FIG. 13 is a rear perspective of an enteral feeding pump;
FIG 14 is a perspective of the support of Fig. 7 shown inside a backpack;
FIG. 15 is a perspective view of another embodiment of a enteral feeding bag support with feet shown in an extended position;
FIG. 16 is a top view of the support of Fig. 15;
FIG. 17 is an enlarged fragmentary view of the support shown in FIG. 16;
FIG. 18 is a bottom view of the support of Fig. 15;
FIG. 19 is an enlarged fragmentary bottom perspective view of the support shown in FIG. 17 with a foot of the support removed to show underlying detail;
FIG. 20 is a perspective view of the enteral feeding bag support of Fig. 15 with the feet shown in a retracted position;
FIG. 21 is a bottom view of the support of Fig. 20;
FIG. 22 is an exploded view of the support of Fig. 15;
FIG. 23 is a perspective of a base of the support of Fig. 22;
FIG. 24 is a perspective of the support of Fig. 20 shown inside a backpack;
FIG. 25 is a perspective view of another embodiment of an enteral feeding bag support with feet shown in a deployed position;
FIG. 26 is a top view of the support of Fig. 25;
FIG. 27 is a bottom view of the support of Fig. 25;
FIG. 28 is an enlarged fragmentary view of the support shown in Fig. 27;
FIG. 29 is a top perspective of a foot of the support of Fig. 25;
FIG. 30 is a bottom perspective of the foot of the support of Fig. 25;
FIG. 31 is an enlarged fragmentary perspective of the support of Fig. 27 with a foot removed;
FIG. 32 is an enlarged fragmentary section of the support;
FIG. 33 is a perspective of the support of Fig. 25 with feet shown in a stowed position;
FIG. 34 is a bottom view of the support of Fig. 33;
FIG. 35 is an exploded view of the support of Fig. 25;
FIG. 36 is a front perspective of a base of the support of Fig. 35;
FIG. 37 is a rear perspective of the base of the support of Fig. 35; and
FIG. 38 is a perspective of the support of Fig. 25 shown inside a backpack.

Corresponding reference characters indicate corresponding parts throughout the several views of the drawings.

### DETAILED DESCRIPTION

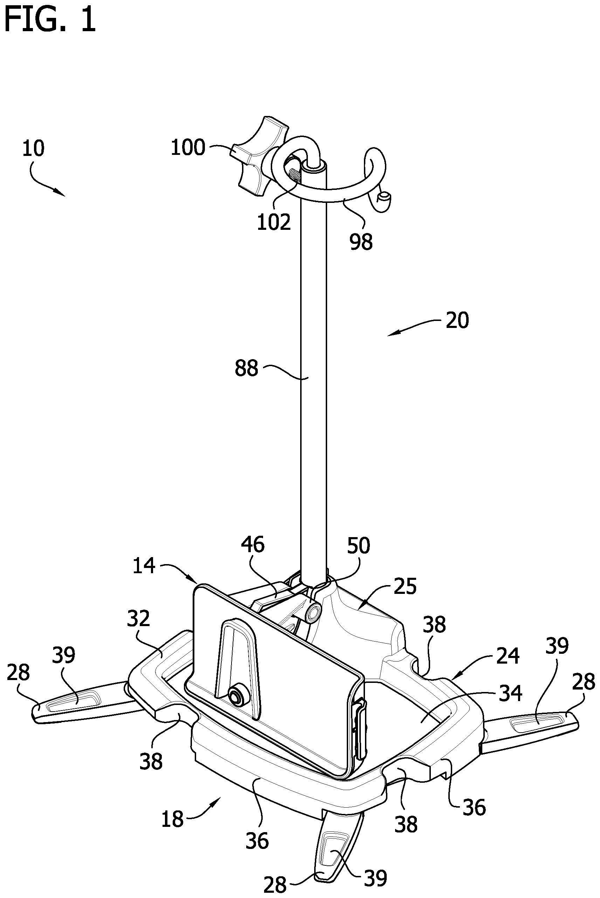

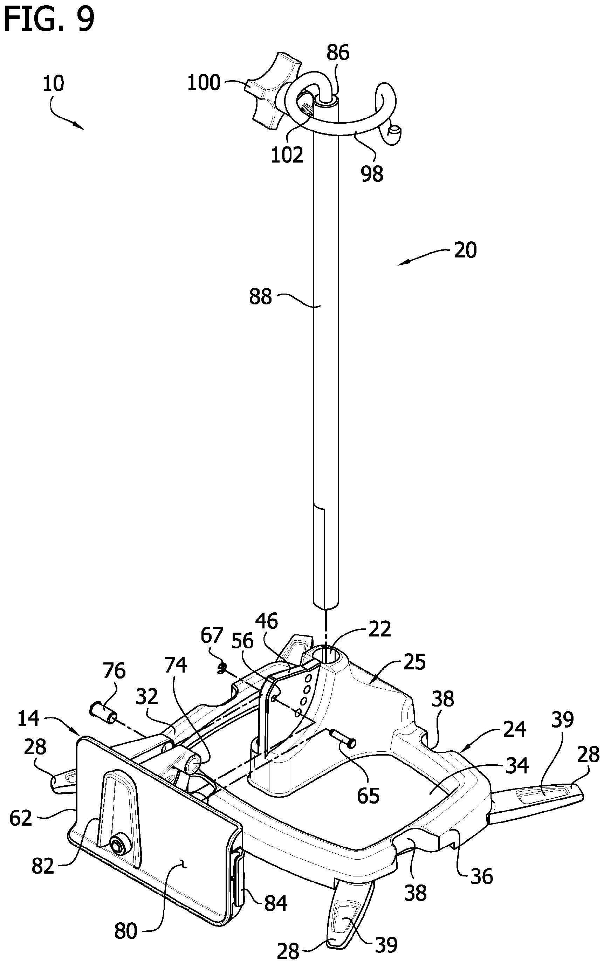

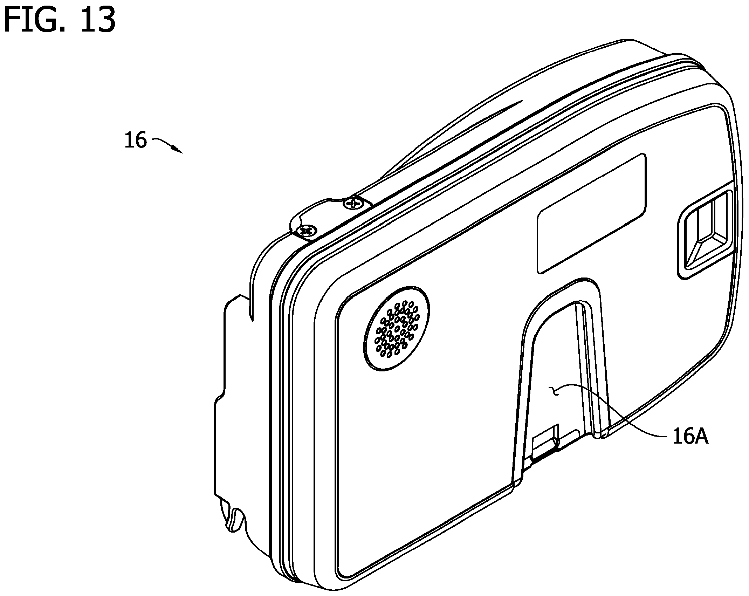

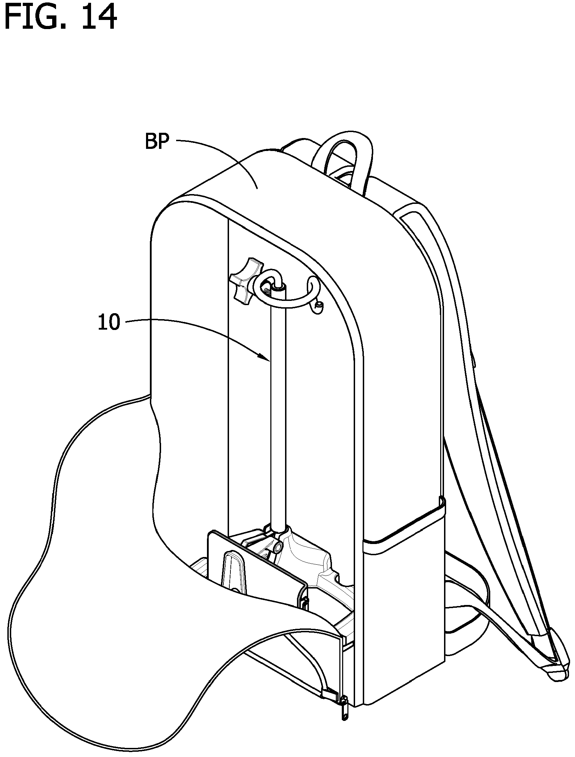

Referring to Figs. 1 and 9, a enteral feeding bag support indicated generally at 10 may be used to suspend a medical bag 12 (Fig. 14), such as an IV or enteral feeding bag, above a support surface, such as a table. The support 10 may also be inserted into a backpack BP to suspend the medical bag 12 in the backpack as shown in Fig. 14, e.g., in an internal compartment thereof. The support 10 may movably attach a medical device mounting assembly 14 for movably and releasably attaching a medical device such as a pump 16 (Figs. 13 and 14) to the support to locate the pump below the medical bag 12 to deliver liquid from the medical bag to a patient.

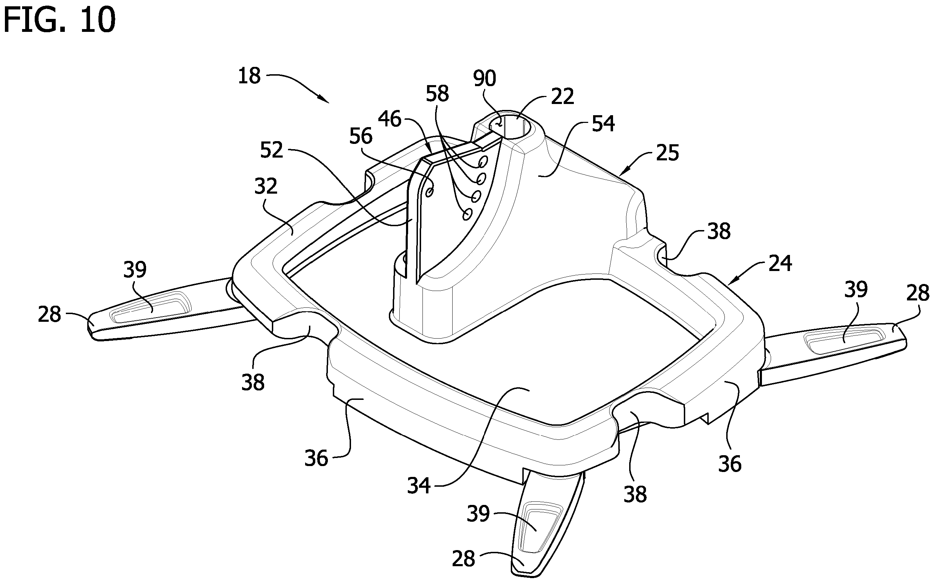

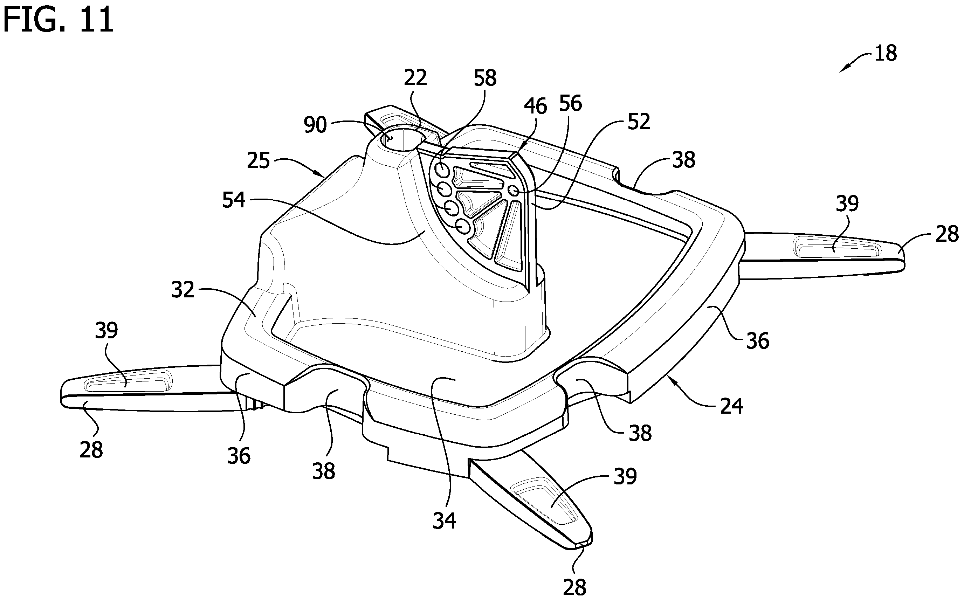

The enteral feeding bag support 10 may comprise a base 18 for supporting the support on the support surface, and a pole assembly 20 configured for attachment to the base to suspend the medical bag 12 above the support surface. The pole assembly 20 may be attached to the base 18 by inserting the pole assembly into an opening 22 (Figs. 10 and 11) in the base and securing the pole assembly in the opening. It is envisioned that the pole assembly 20 can be secured to the base 18 is other ways without departing from the scope of the disclosure. Suitable structure (not shown) may be provided to secure the pole assembly 20 to the base 18 and/or to secure the pole assembly against rotation relative to the base.

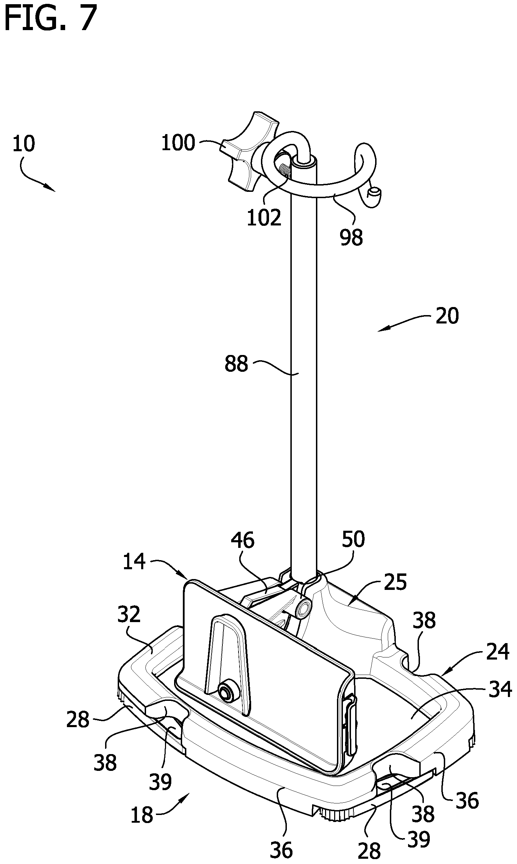

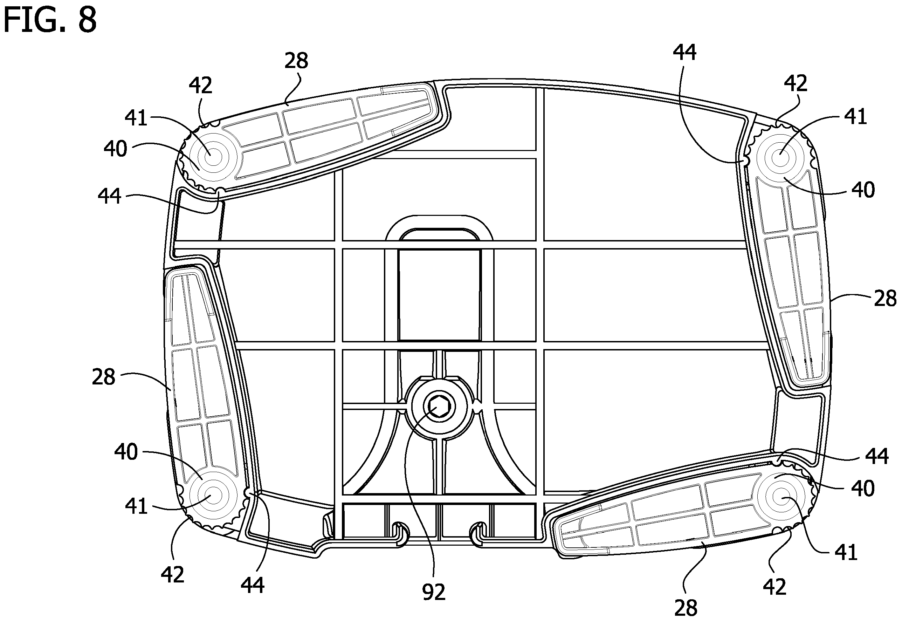

Referring to Figs. 1-8, the base 18 may comprise a support portion 24 for supporting the support 10 on the support surface and an attachment portion 25 for attaching the pole assembly 20 to the base and movably attaching the medical device mounting assembly 14 to the base. Feet 28 may be attached to a bottom of the support portion 24 for engaging the support surface to stabilize the support 10 on the support surface. In the illustrated embodiment, each foot 28 is pivotably attached to the bottom of the support portion 24 and movable between a deployed position (Fig. 1) and a stowed position (Figs. 7 and 8). In the stowed position, the feet 28 are received in pockets 30 in the bottom of the support portion 24 such that the feet are substantially within an outer perimeter of the support portion. In the deployed position the feet 28 may extend outward past the outer perimeter of the support portion 24 to provide a wider support base for the support 10. The feet 28 could be fixedly attached to the support portion 24 without departing from the scope of the disclosure. In the illustrated embodiment, there are four feet 28 attached to the support portion 24. However, any number of feet is envisioned.

The support portion 24 may have a rim 32 and a floor 34 recessed from the rim. The rim 32 and floor 34 define a tray of the support portion 24 which can be used to hold items associated with the use of the support 10. The rim 32 may have other constructions without departing from the scope of the disclosure. Sides 36 of the support portion 24 may have cutouts 38. In the illustrated embodiment, one cutout 38 is associated with each side 36. A portion of each cutout 38 may be aligned with at least a portion of a respective foot 28 when the foot is in the stowed position. The cutouts 38 provide access to the feet 28 from a side and/or from above the support portion 24 so that the feet can be grasped and moved to the deployed position when the support 10 is supported on a support surface. A recessed portion 39 in a top of each foot 28 may provide an area to grip the foot to facilitate grasping the foot and moving it from the stowed position to the deployed position.

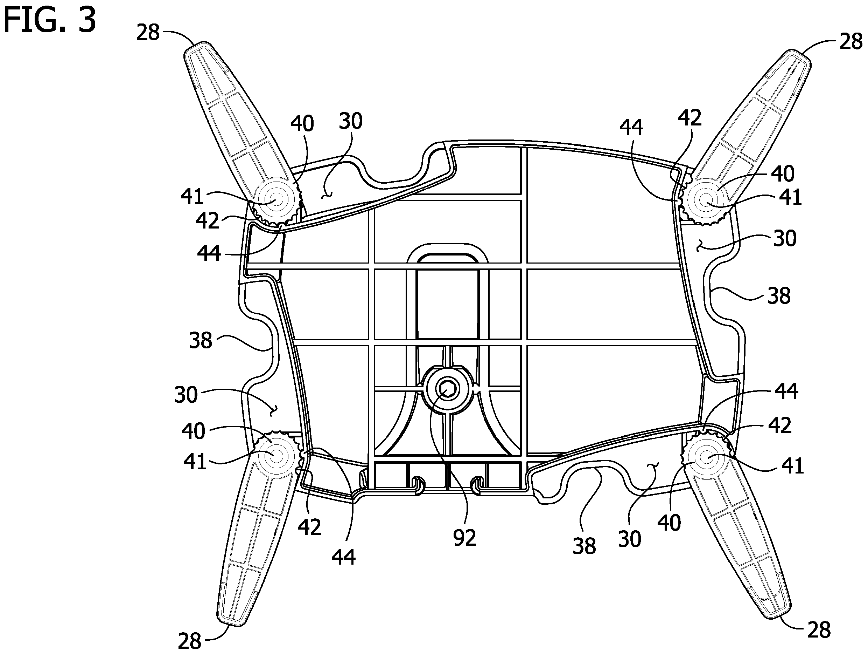

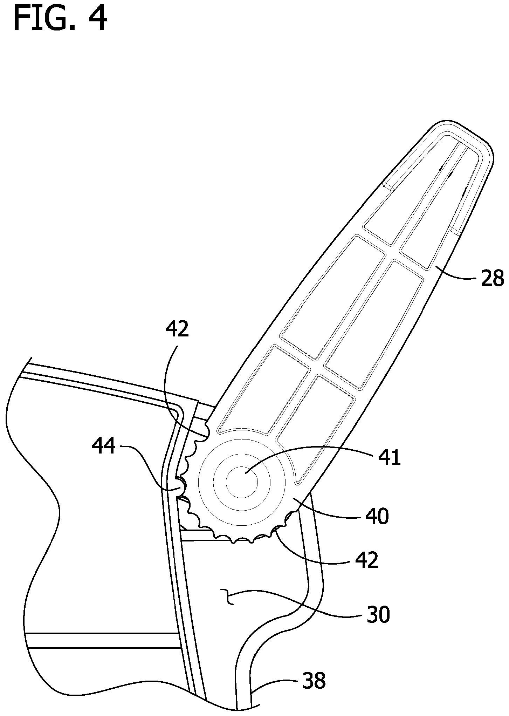

Referring to Figs. 3 and 4, each foot 28 may include an anchor portion 40 that is pivotably attached to the bottom of the support portion 24 by a fastener 41. The anchor portion 40 may have a plurality of indentations 42 formed around an outer surface of the anchor portion 40. A detent 44 may be formed on an inner surface of each pocket 30 and configured to be removably received in the indentations 42 to releasably hold the foot 28 in any of several positions. At least one of the anchor portion 40 and the detent 44 is resiliently deformable to permit movement of the foot from the fully stowed to the fully deployed position and back. It will be understood that the detent 44 holds the foot 28 in both the fully stowed and fully deployed positions.

Referring to Figs. 1, 2, 10, and 11, the attachment portion 25 of the base 18 may comprise an attachment member 46 and the attachment portion may be formed integrally with the support portion 24. The attachment portion 25 may be disposed generally at the back of the support portion 24 and may extend upward from the support portion. The attachment member 46 may be fixedly attached to a remainder of the attachment portion 25. In the illustrated embodiment, the attachment member is formed as one piece with the remainder of the attachment portion 25. The attachment member 46 can be formed in other ways without departing from the scope of the disclosure.

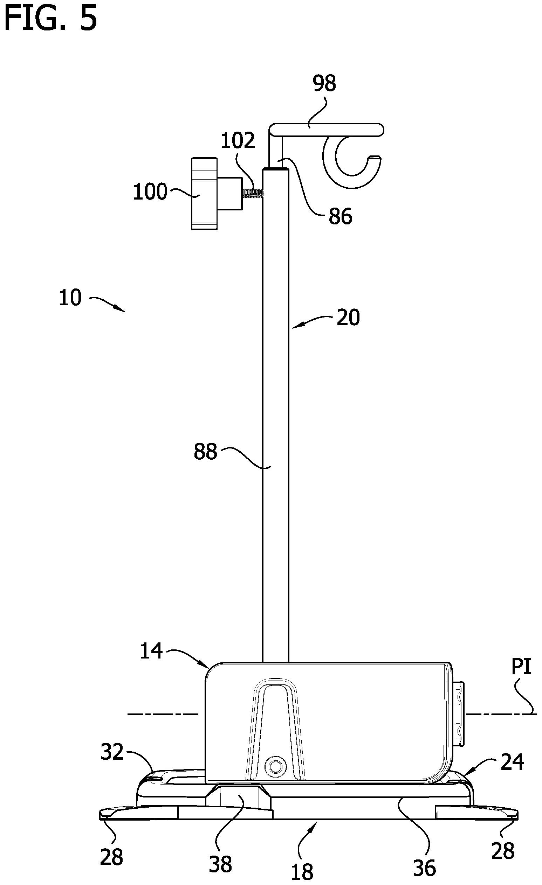

The attachment member 46 may pivotally attach a mounting portion 50 of the medical device mounting assembly 14 to the attachment portion 25 of the base 18. The attachment member 46 may comprise a tab 52 projecting from an arcuate section 54 of the attachment portion 25. The tab 52 may include a pivot hole 56 generally at a front end of the tab and a plurality of locking holes 58 arcuately spaced from one another and extending generally along a back end of the tab adjacent the arcuate section 54 of the attachment portion 25. The locking holes 58 are arranged at an approximately constant distance from the pivot hole 56. The locking holes 58 may be arranged to provide about 45 degrees of pivot adjustment between the mounting portion 50 and the attachment member 46 about a pitch axis PI (Fig. 5) extending through the pivot hole 56. In the illustrated embodiment, the pivot hole 56 is disposed at a top of the front end of the tab 52, and a topmost locking hole 58 is disposed near a top of the back end of the tab. It is envisioned that the attachment member 46 may have other shapes and the pivot hole 56 and locking holes 58 may be disposed in other locations.

Referring to Fig. 14, the support 10 may be carried in a backpack BP within an internal compartment thereof such that a medical bag 12 is suspended by the support inside the backpack. The feet 28 of the support 10 may be moved to the stowed position (Fig. 7) to facilitate inserting the support into the backpack BP. However, the feet 28 may be moved to the deployed position (Fig. 1) and inserted into the backpack BP without departing from the scope of the disclosure. The backpack BP allows the patient the freedom to be mobile while using the support 10 and pump 16 mounted on the support. The same support 10 can be used for table top support out of the backpack BP by moving the feet 28 to the deployed position for stability.

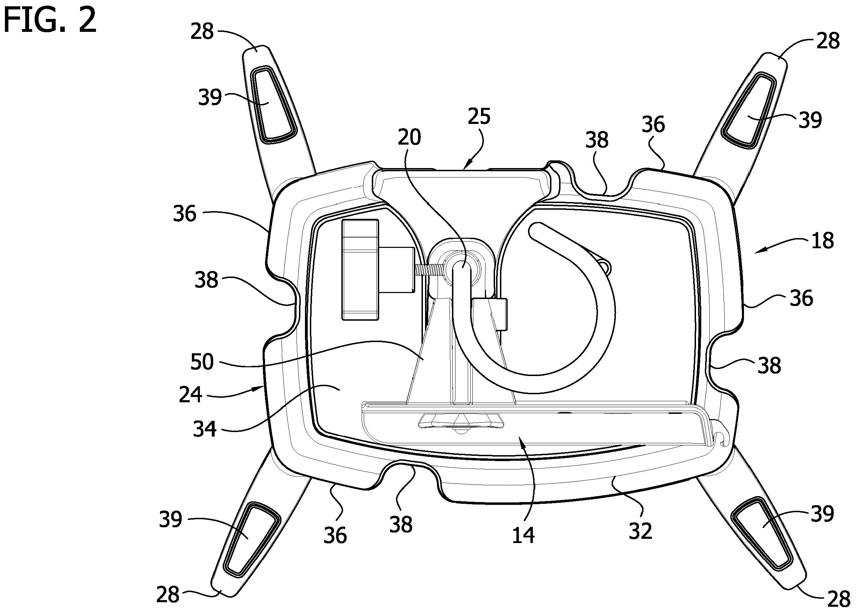

Referring to Figs. 1, 2, 9, and 12, the medical device mounting assembly 14 may comprise the mounting portion 50 for attaching to the attachment member 46 of the attachment portion 25, and a device receiving platform 62 for releasably attaching a medical device, such as the enteral feeding pump 16 shown in Fig. 13. The pump 16 can be mounted so that it can be tilted up or down as is convenient for viewing and/or manipulation. The mounting portion 50 may comprise a pair of spaced apart arms 64A, 64B defining a channel 66 sized to receive at least a portion of the tab 52 of the attachment member 46. The arms 64A, 64B may have aligned holes (not shown) near respective bases of the arms for receiving a pivot pin 65 to mechanically link the mounting portion 50 to the attachment member 46 for rotation about the pitch axis PI. The pivot pin 65 may be secured in the pivot hole 56 and aligned holes by a C-clip 67. In particular, the pivot pin 65 may extend through the pivot hole 56 in the attachment member 46 and the aligned holes in the mounting portion 50 to pivotally link the medical device mounting assembly 14 to the attachment member. This connection allows the mounting assembly 14 to pivot relative to the attachment member 46 about the pitch axis PI. The channel 66 may have a width only slightly larger than a thickness of the tab 52 to substantially inhibit movement of the mounting portion 50 relative to the tab along a single pivot axis P1 (Figs. 2 and 5) .

A detent hole 74 may be disposed near the top of arm 64A for receiving a locking detent 76 (Fig. 9). The locking detent may be adapted for selective engagement with the locking holes 58 in the attachment member 46. The detent 76 may also be spring loaded such that a tip of the detent is movable relative to a base of the detent. When the detent 76 is received in one of the locking holes 58 and a torque of magnitude more than a threshold above the torque applied by the weight of the medical device mounting assembly 14 plus the weight of the enteral feeding pump 16 (when attached thereto) is applied to the mounting portion, a lip of the locking hole 58 may engage the tip of the detent 76 and move the tip of the detent into the base providing a clearance for the device attachment portion to pivot relative to the attachment member 46. Upon coming into registration with an adjacent locking hole 58, the spring loaded detent 76 may urge the tip into the hole to again releasably lock the mounting portion 50 in place on the attachment member 46. The locking detent 76 may have other configurations allowing the detent to releasably engage the locking holes 58 in the attachment member 46.

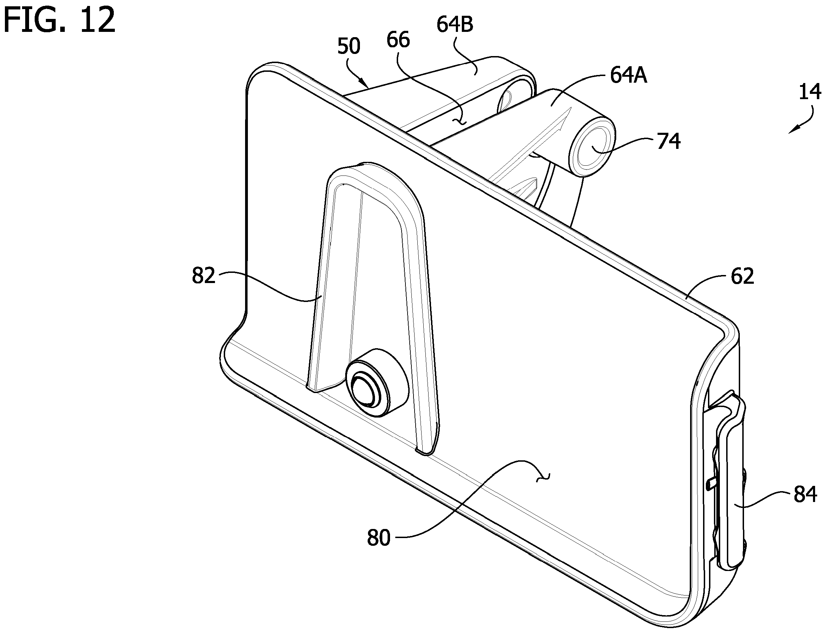

The device receiving platform 62 of the medical device mounting assembly 14 may form a pocket 80 (Fig. 12). A device mount 82 may be disposed on a rear wall of pocket 80 releasably attaching the enteral feeding pump 16 to the support 10 by being removably received in a recess 16A on the back of the pump. A tube holder 84 may be disposed on a side wall of the pocket 80 to retain medical tubing extending to or from the pump 16.

In the illustrated embodiments, the pole assembly 20 has a selectively adjustable height. Referring to Figs. 1-3 and 5-8, the pole assembly 20 may be inserted into the opening 22 in the attachment portion 25 of the base 18. The pole assembly 20 may comprise an inner tube member 86 and an outer tube member 88 surrounding at least a portion of the inner tube member. At least the outer tube member 88 extends into the opening 22 in the base 18. However, in at least one configuration both the outer tube member 88 and the inner tube member 86 extend into the opening 22. In the illustrated embodiment, the outer tube member 88 extends through the opening 22 and into a passage 90 in the base 18. The passage 90 extends from the opening 22 in the top of the attachment portion 18 to the bottom of the support portion 24. The outer tube member 88 may be secured in the passage 90 with a screw 92 (Figs. 3 and 8) inserted through the base 18 into the passage 90 at the bottom of the support portion 24. The screw 92 may engage a threaded bottom portion (not shown) of the outer tube member 88 to secure the outer tube member in the passage 90.

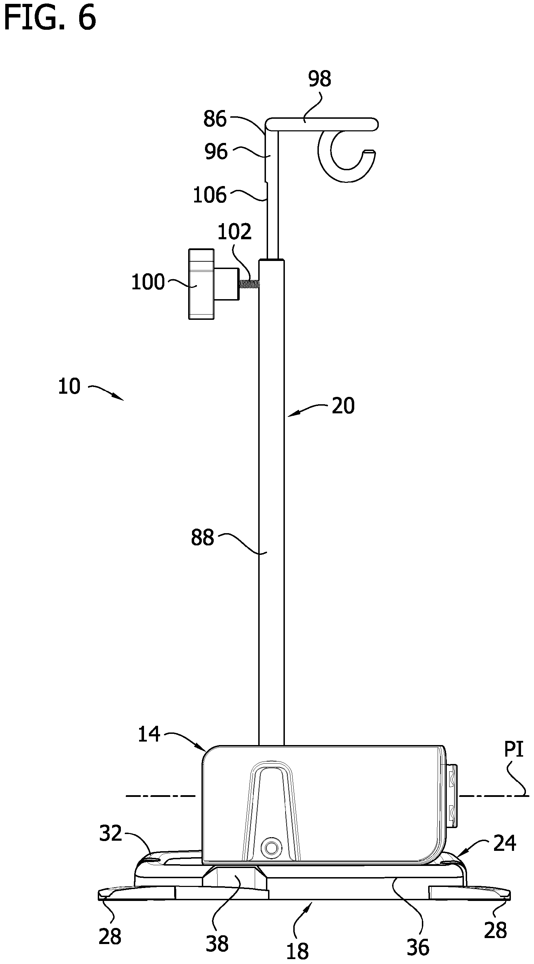

The inner tube member 86 may include a first straight section 96 (Fig. 6) received in the outer tube member 88 and a second curved section 98 extending from the first straight section for attaching the medical bag 12 to the pole assembly 20. When the medical bag 12 is attached to the second curved section 98 and the base 18 is supported on a support surface, the medical bag is suspended above the support surface by the pole assembly 20. The inner tube member 86 is slidably received in the outer tube member 88 for adjusting a height and position of the second curved section 98 relative to the support surface. A knob 100 and screw 102 can lock the inner tube member 86 at a selected height and position relative to the support surface. Locking the inner tube member 86 in place can be achieved by rotating the knob 100 in a locking direction to advance the screw 102 in a threaded hole in the outer tube member 88 to engage the screw with a flat surface 106 (Fig. 6) of the inner tube member to secure the inner tube member at a selected height and position. To unlock the inner tube member 86 to adjust its relative height and position, the knob 100 is rotated in an unlocking direction to disengage the screw 102 with the inner tube member to allow the inner tube member to freely slide relative to the outer tube member 88. The relative height and position of the inner tube member 86 can be adjusted and locked in other ways without departing from the scope of the disclosure.

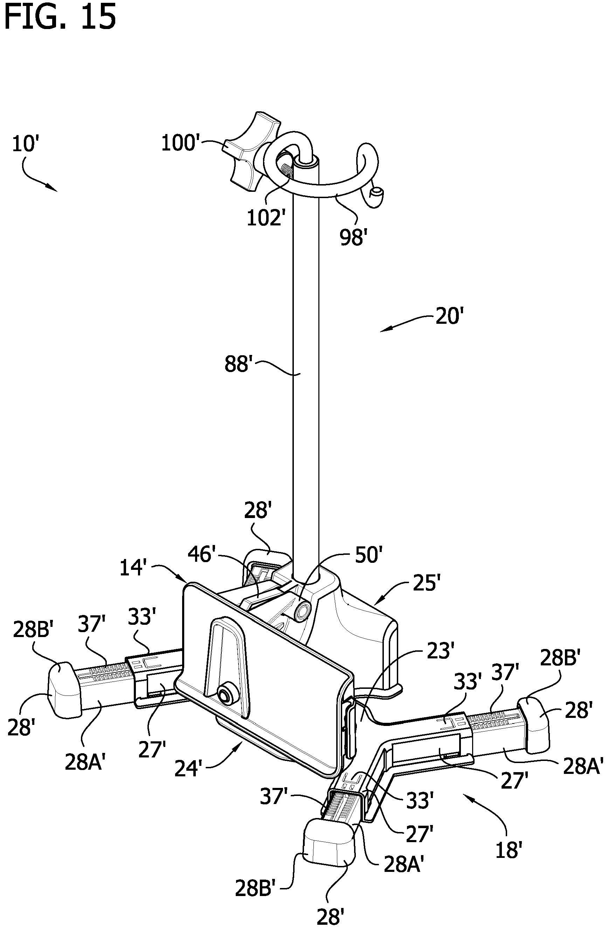

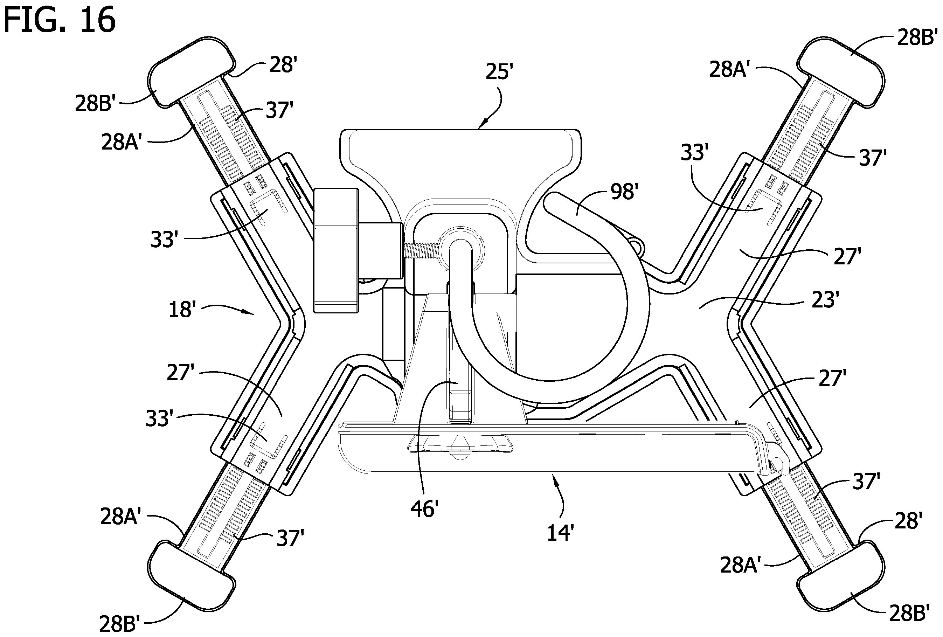

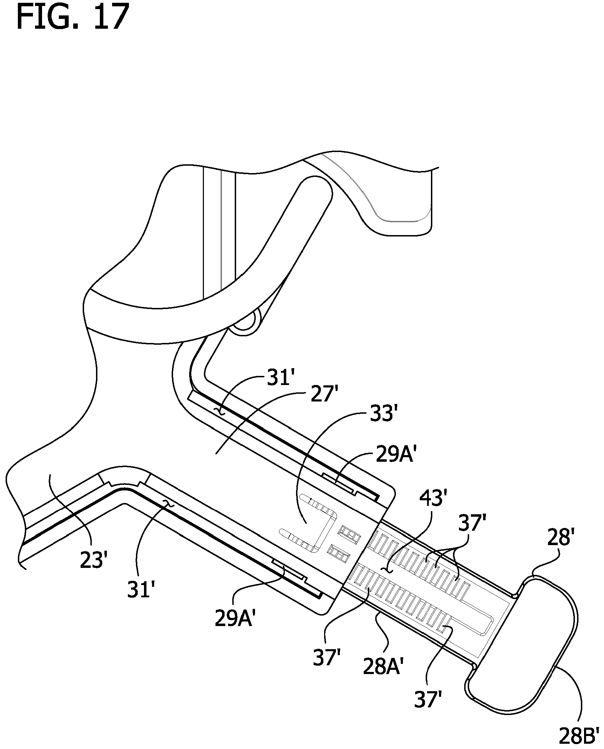

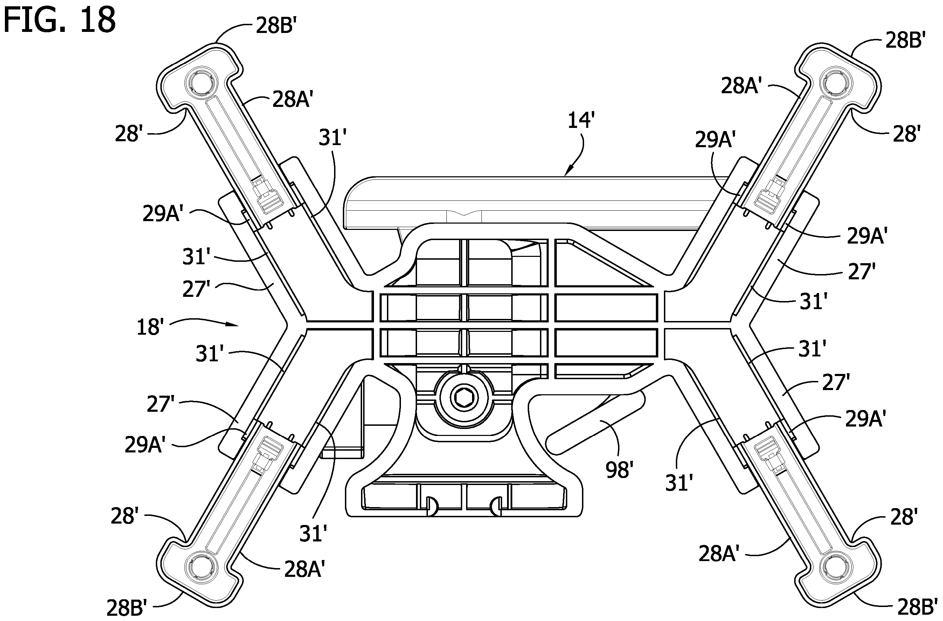

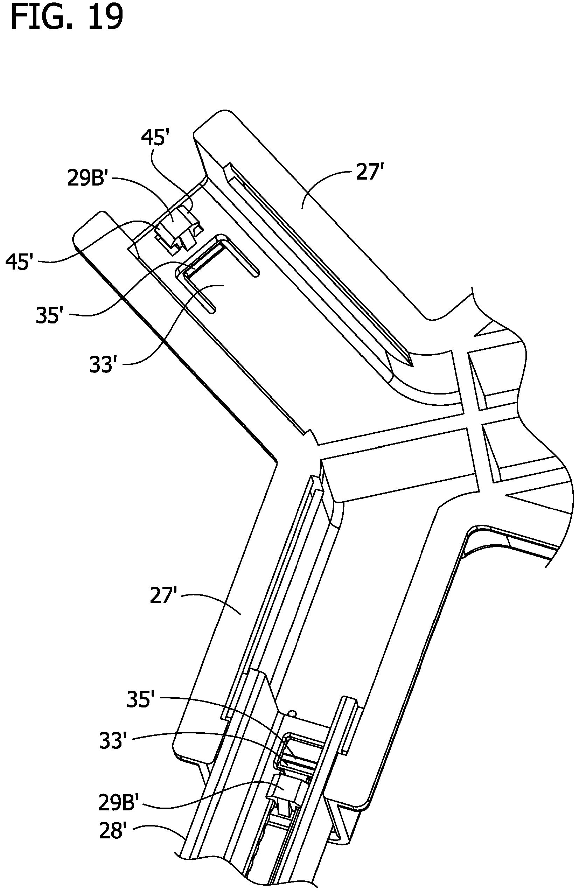

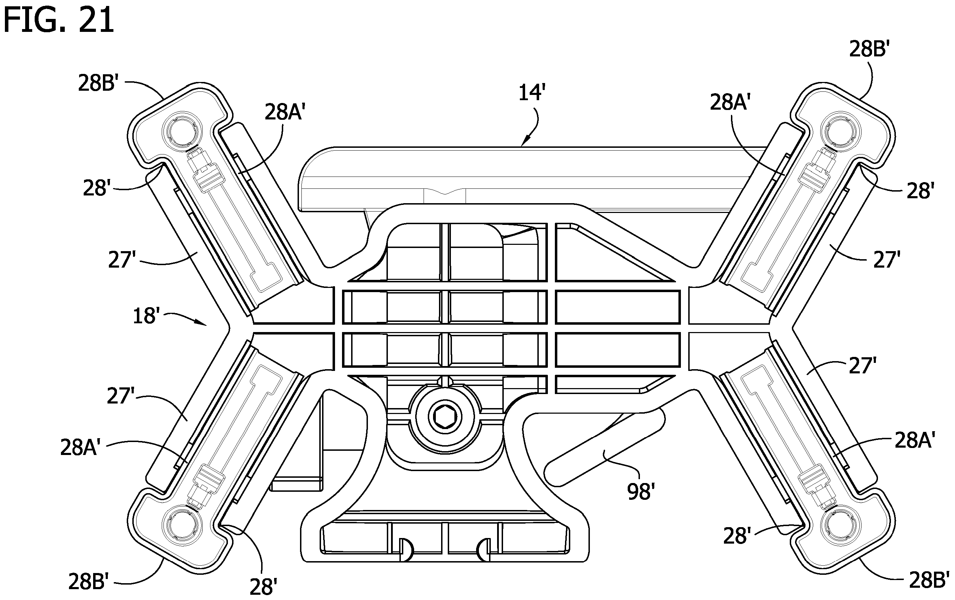

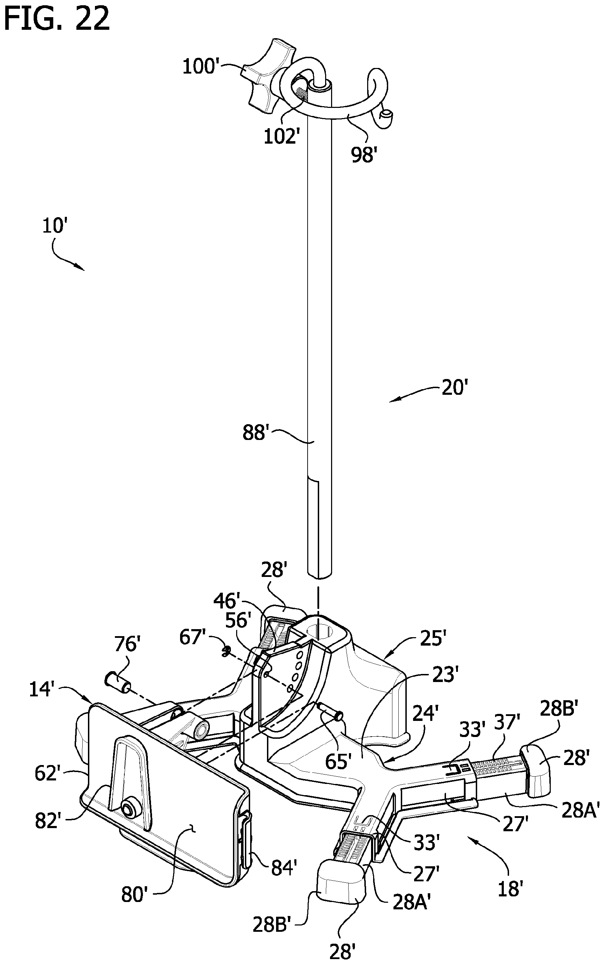

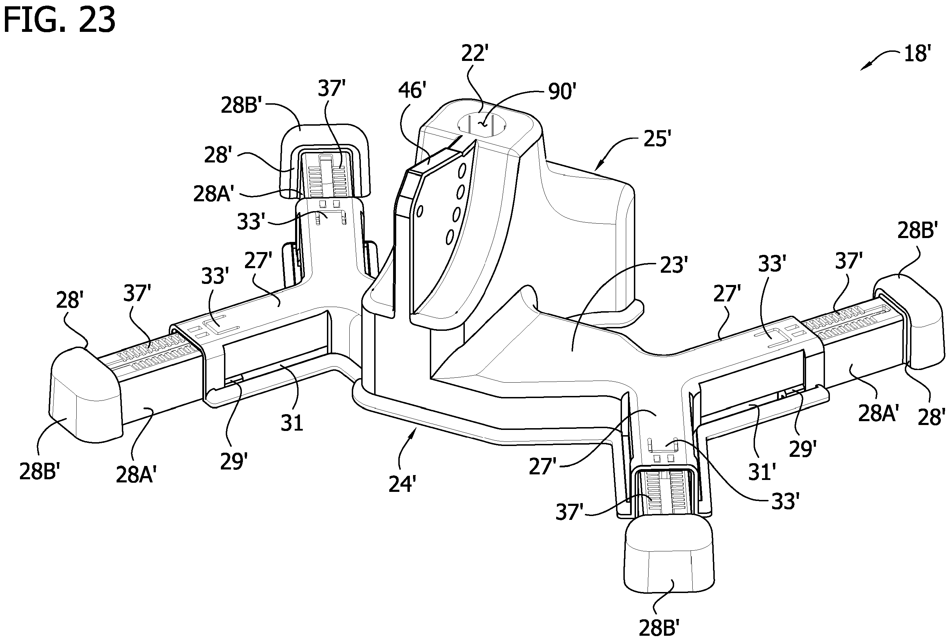

Referring to Figs. 15-24, an enteral feeding bag support of a second embodiment is indicated generally at 10'. The support 10' of the second embodiment is similar to the support 10 of the first embodiment except as described hereinafter. Accordingly, like components are indicated by corresponding reference numerals followed by a prime. The support 10' may comprise a base 18' for resting on a surface (not shown), and a pole assembly 20' configured for attachment to the base to suspend a medical bag (not shown) above the support surface. The base 18' may comprise a support portion 24' for supporting the support 10' on the support surface and an attachment portion 25' for attaching the pole assembly 20' to the base and movably attaching a medical device mounting assembly 14' to the base. The support portion 24' may be configured to engage the support surface. The support portion 24' may include a body 23' and legs 27' extending from the body. Feet 28' may be attached to the legs 27' for engaging the support surface to stabilize the support 10' on the support surface. The feet 28' may each include a first portion 28A', a least a portion of which is received in a respective leg 27' in telescoping fashion. A second portion 28B' of the foot 28' is wider than the first portion 28A' and disposed on an end of the first portion. The wider second portion 28B' can provide added stability to the support 10'.

Referring to Figs. 15-20, each foot 28' is slidably attached to a respective leg 27' of the support portion 24' and movable along a longitudinal axis of the leg between retracted and extended positions. The sliding movement of each foot 28' may be guided through the engagement of first slide members 29A' disposed on the first portion 28A' of the foot 28' and channels 31' formed in the legs 27'. As the feet 28' are moved relative to the legs 27', the first slide members 29A' slide along the channels 31' guiding the movement of the feet. A second slide member 29B' on the legs 27' slides along a channel 43' in the first portion 28A' of the feet 28'. The second slide member 29B' is retained in the channel 43' by hooks 45' on the second slide member. The engagement between the second slide members 29B' and the channels 43' also guides the sliding movement of the feet 28' relative to the legs 27'. In the illustrated embodiment, the hooks 45' are formed integrally with a remainder of the second slide member 29B'. The hooks 45' could be formed separately from the second slide member 29B' and attached to the second slide member by suitable means.

Each foot 28' can be selectively and releasably locked in incremental positions along the longitudinal axes of the legs 27'. In the illustrated embodiment, the legs 27' each have tabs 33' including a rounded projection 35' configured to releasably engage in any one of slots 37' in the feet 28'. A pulling or pushing force of a sufficient amount on one of the feet 28' along the longitudinal axis of the respective leg 27' can cause the tab 33' on the leg to resiliently flex as the projection 35' engages an edge of a slot 37' in the foot moving the projection out of the slot and into an adjacent slot 37'. Each foot 28' can be individually pushed or pulled to place the foot in a selected position. The feet 28' could be fixedly attached to the support portion 24' without departing from the scope of the disclosure. In the illustrated embodiment, there are four feet 28' attached to the support portion 24'. However, any number of feet is envisioned.

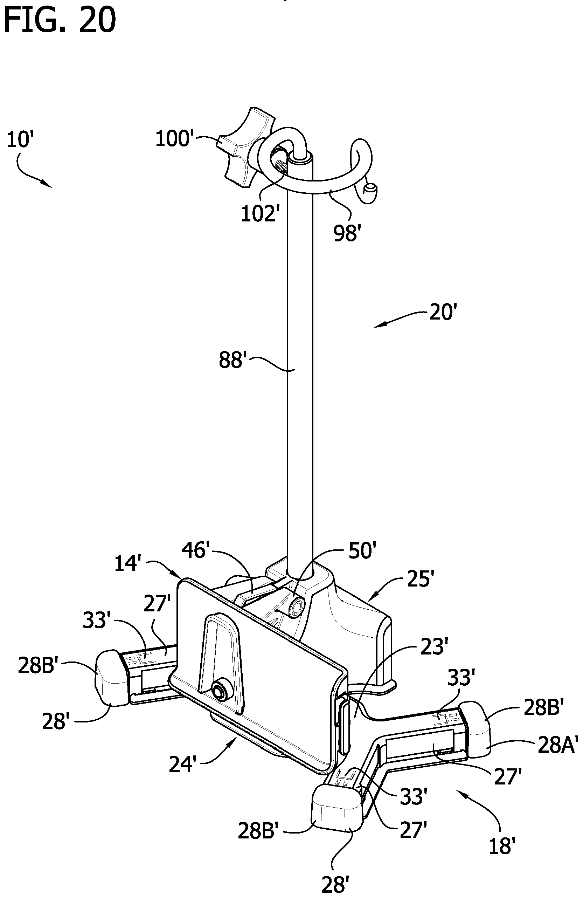

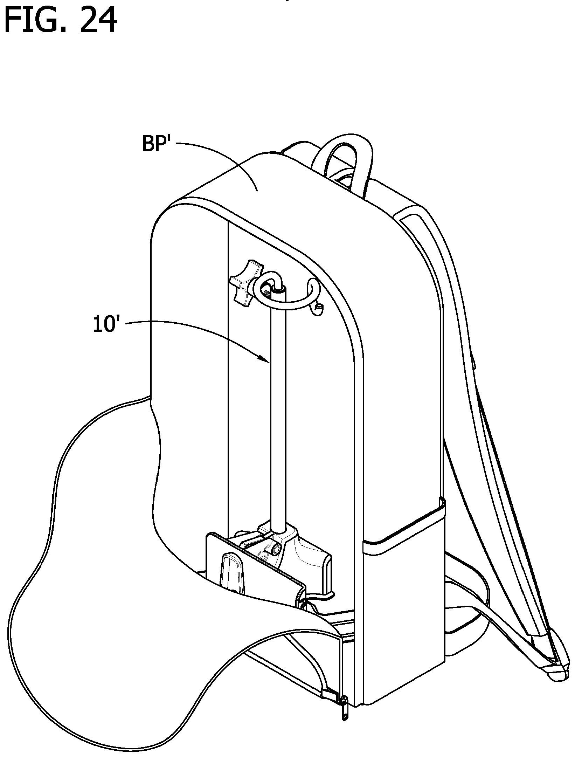

Referring to Fig. 24, the support 10' may be carried in a backpack BP such that a medical bag (not shown) is suspended by the support inside the backpack. The feet 28' of the support 10' may be moved to the retracted position (Fig. 20) to facilitate inserting the support into the backpack BP. However, the feet 28' may be moved to the deployed position (Fig. 15) and inserted into the backpack BP without departing from the scope of the disclosure. The backpack BP allows the patient the freedom to be mobile while using the support 10' and a pump mounted on the support.

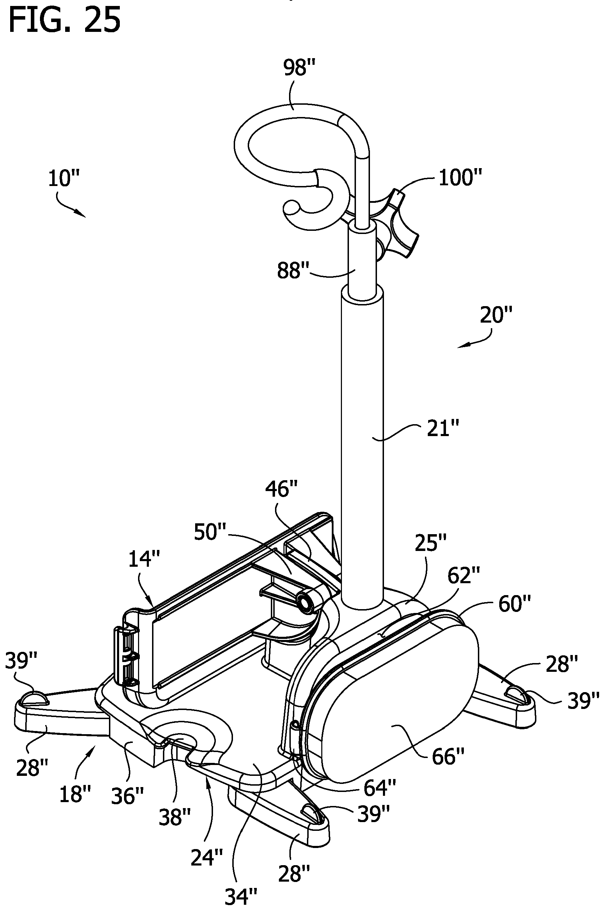

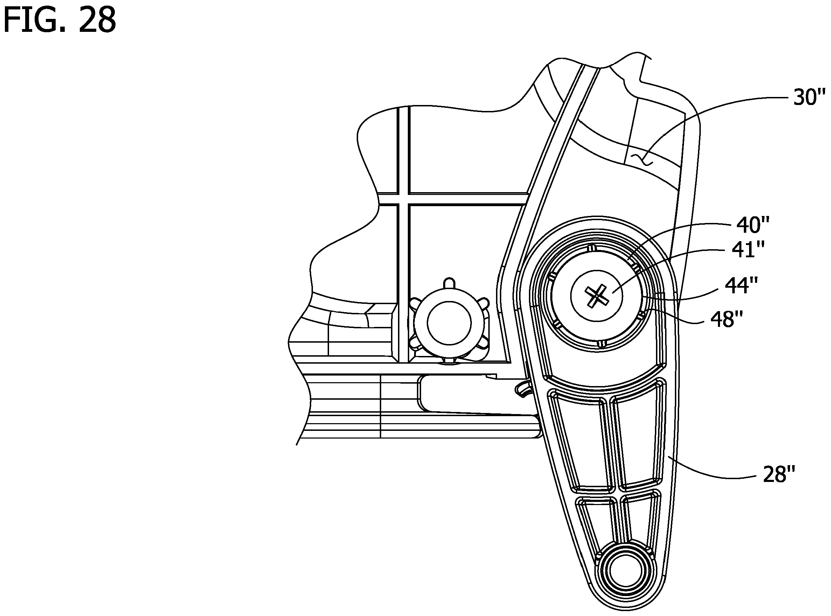

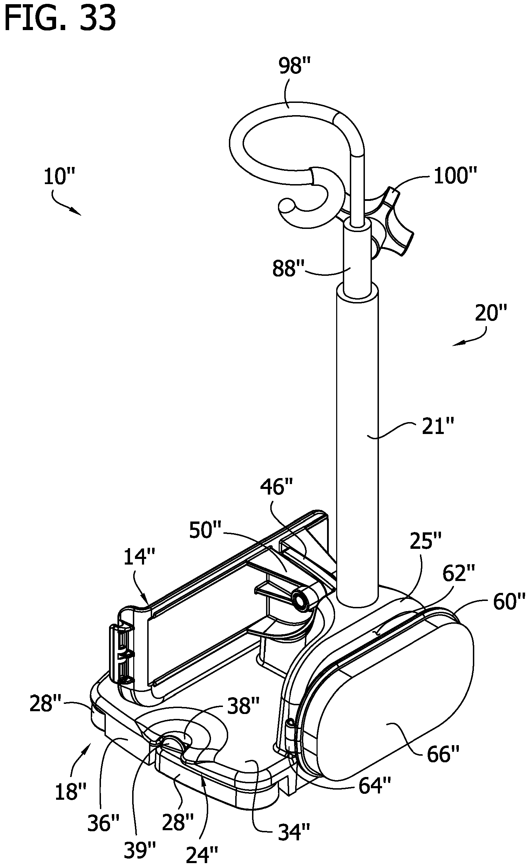

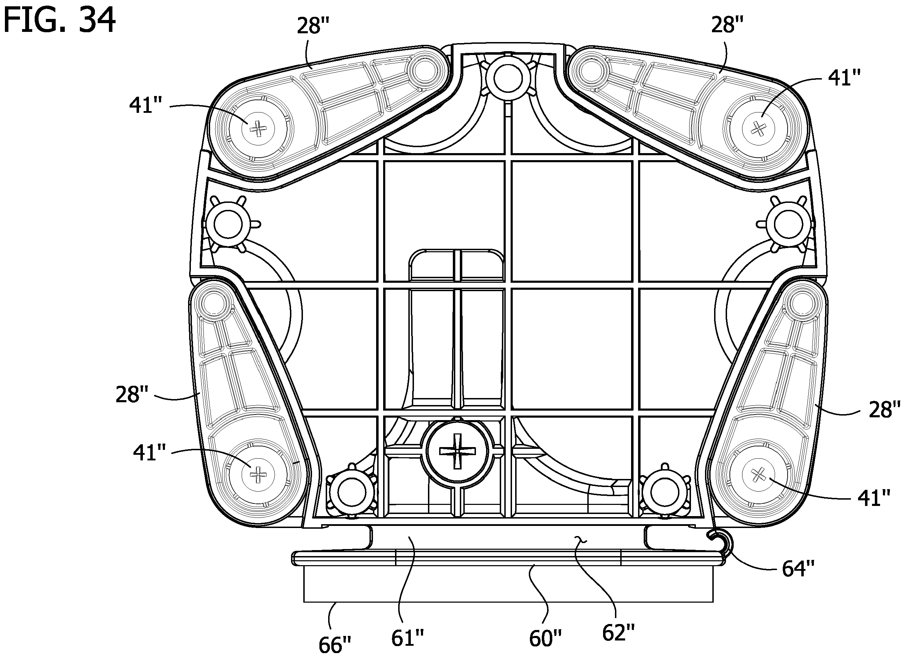

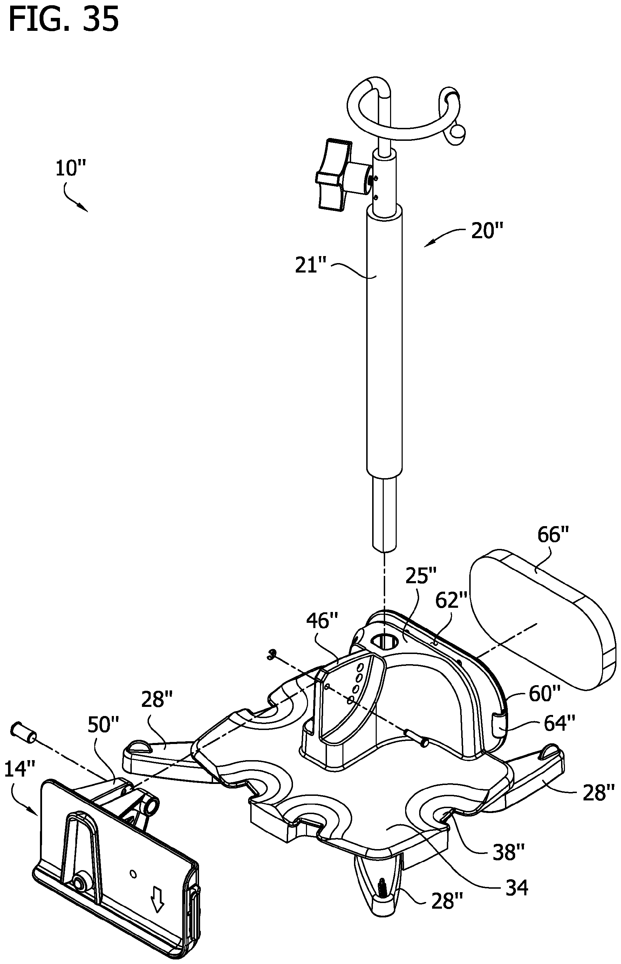

Referring to Figs. 25-38, an enteral feeding bag support of a third embodiment is indicated generally at 10". The support 10" of the third embodiment is similar to the support 10 of the first embodiment except as described hereinafter. Accordingly, like components are indicated by corresponding reference numerals followed by a double prime. The support 10" may comprise a base 18" for resting on a surface (not shown), and a pole assembly 20" configured for attachment to the base to suspend a medical bag (not shown) above the support surface. The pole assembly 20" may include padding 21". The base 18" may comprise a support portion 24" for supporting the support 10" on the support surface and an attachment portion 25" for attaching the pole assembly 20" to the base and movably attaching a medical device mounting assembly 14" to the base. The support portion 24" may be configured to engage the support surface. Feet 28" may be attached to a bottom of the support portion 24" for engaging the support surface to stabilize the support 10" on the support surface. In the illustrated embodiment, each foot 28" is pivotably attached to the bottom of the support portion 24" and movable between a deployed position (Fig. 25) and a stowed position (Figs. 33 and 34). In the stowed position, the feet 28" are received in pockets 30" in the bottom of the support portion 24" so that the feet are substantially within an outer perimeter of the support portion. In the deployed position the feet 28" may extend outward past the outer perimeter of the support portion 24" to provide a wider support base for the support 10". The feet 28" could be fixedly attached to the support portion 24" without departing from the scope of the disclosure. In the illustrated embodiment, there are four feet 28" attached to the support portion 24". However, any number of feet is envisioned.

The support portion 24" may have a top surface 34". Sides 36" of the support portion 24" may have cutouts 38". In the illustrated embodiment, there are two cutouts 38" in a front side 36" of the support 10", and one cutout 38" in each of the lateral sides 36" of the support. A portion of each cutout 38" may be aligned with at least a portion of a respective foot 28" when the foot is in the stowed position. The cutouts 38" provide access to the feet 28" from a side and/or from above the support portion 24" so that the feet can be grasped and moved to the deployed position when the support 10" is supported on a support surface. A tab 39" on a top of each foot 28" may provide structure to facilitate grasping the foot and moving it between the stowed position and the deployed position.

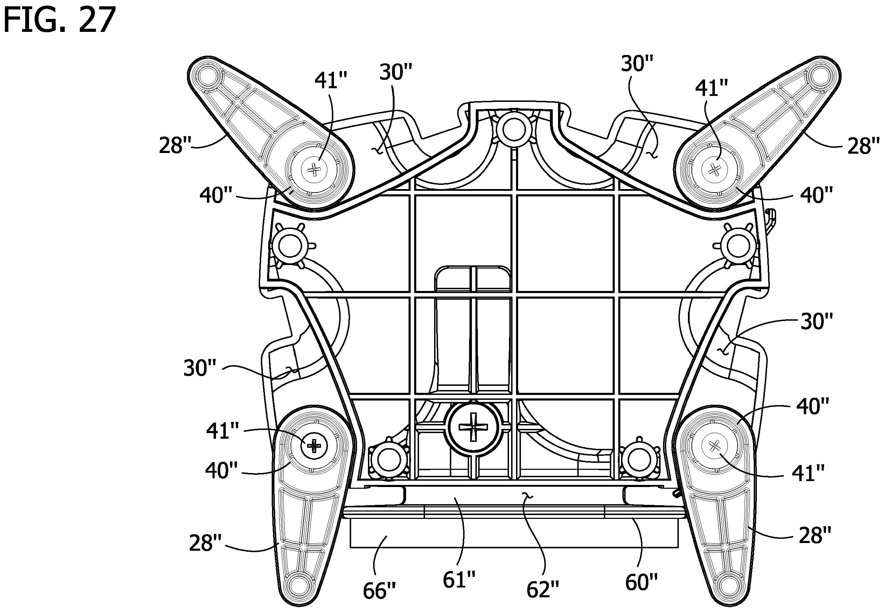

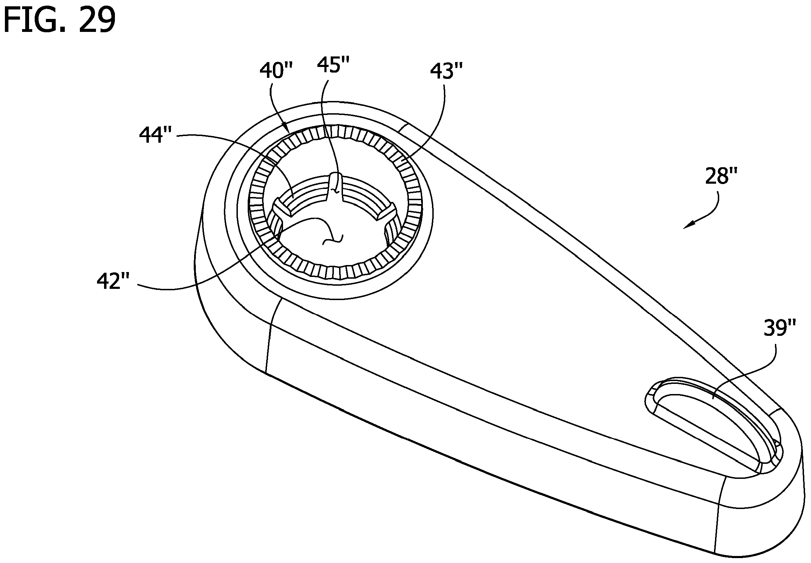

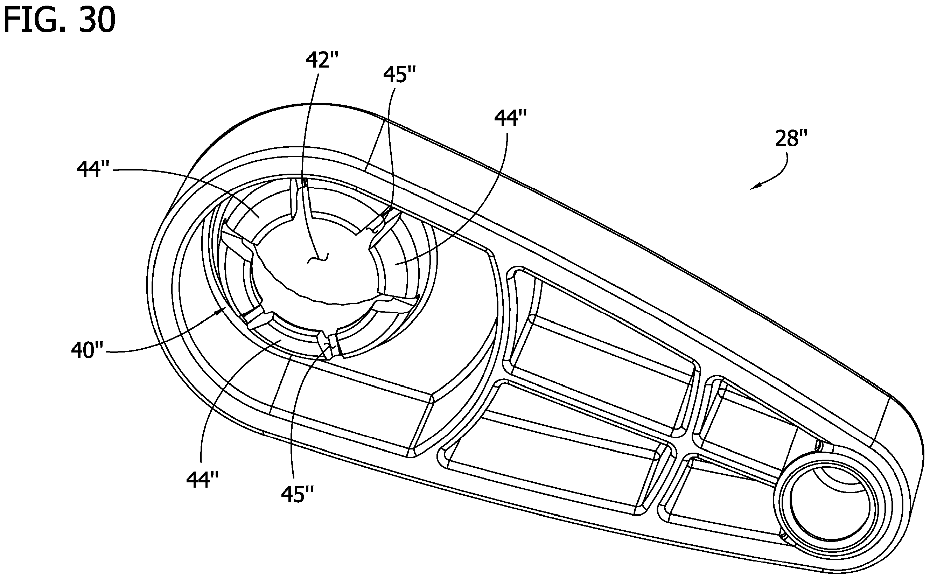

Referring to Figs. 27-32, each foot 28" may include an anchor portion 40" that is pivotably attached to the bottom of the support portion 24" by a fastener 41". The anchor portion 40" may have an opening 42" (Figs. 29 and 30) and a plurality of notches 43" formed in a top surface of the anchor portion 40" around the opening. A plurality of flanges 44" extend from a bottom surface of the anchor portion 40" partially across the opening 42". The flanges 44" may comprise discrete flexible members defining gaps 45" between adjacent flanges.

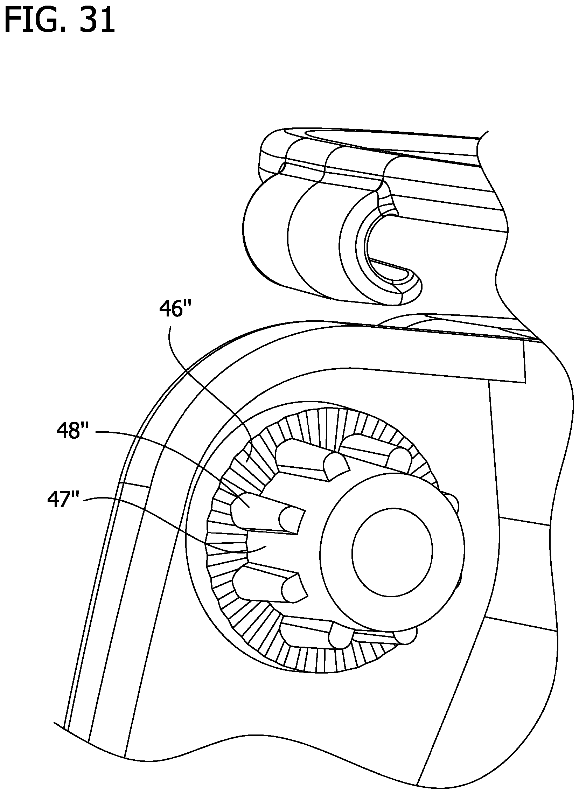

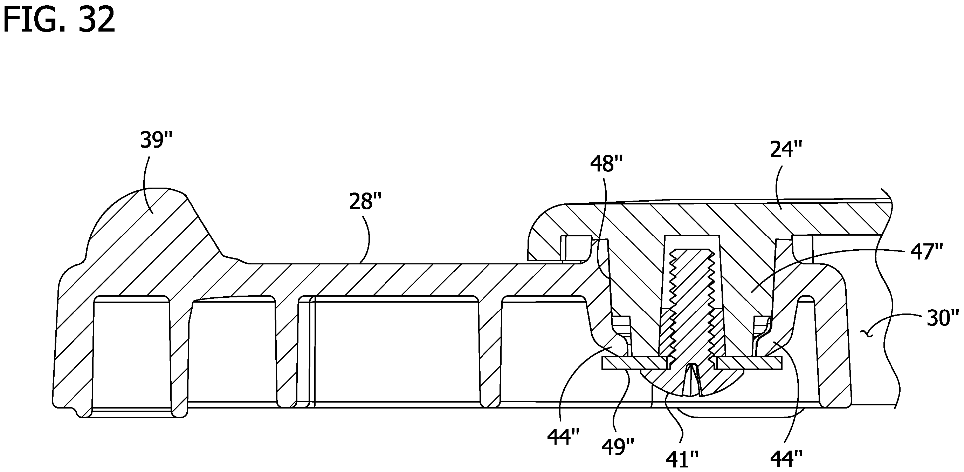

Detents 46" (Fig. 31) may be formed on a bottom surface of an associated pocket 30" in the support portion 24" of the base 18". A post 47" may extend from the bottom surface of the pocket 30" and ribs 48" may be formed on an outer surface of the post 47". The post 47" is received in the opening 42" in the foot 28" and the foot is secured to the support portion 24" by the fastener 41". The detents 46" in the pocket 30" may be configured to be removably received in the notches 43" on the foot 28", and the ribs 48" on the post 47" may be configured to engage a surface extending around the opening 42" to releasably hold the foot in any of several positions with respect to the support portion 24". Pivoting the foot 28" causes the notches 43" on the foot to move relative to the detents 46". For the notches 43" to clear the detents 46" during rotation of the foot 28", the anchor portion 40" of the foot must move away from the support portion 24" to provide the necessary clearance. The angled surfaces on the notches 43" and detents" urge the foot 28" away from the support portion 24" when a rotational force is applied to the foot. To allow for this separation, the flanges 44" on the foot 28" engage a washer 49" of the fastener 41" and flex upward allowing the notches 43" to slide down the detents 46" to at least partially disengage from the detents (Fig. 32) . Once the notches 43" clear the detents 46", the foot 28" can be rotated to a desired position. After rotating the foot 28" to the desired position, the foot is released and the flanges 44" will urge the notches 43" back into engagement with the detents 46" and hold the foot in the new position. It will be understood that the engagement between the detents 46" and notches 43", the engagement between the ribs 48" and the surface around the opening 42", and the engagement between the flanges 44" and the washer 49" hold the foot 28" in both the fully stowed and fully deployed positions.

Referring to Figs. 25 and 35-37, the attachment portion 25" of the base 18" may comprise an attachment member 46" and the attachment portion may be formed integrally with the support portion 24". The attachment portion 25" may be disposed generally at the back of the support portion 24" and may extend upward from the support portion. The attachment member 46" may be fixedly attached to a remainder of the attachment portion 25". In the illustrated embodiment, the attachment member 46" is formed as one piece with the remainder of the attachment portion 25". The attachment member 46" can be formed in other ways without departing from the scope of the disclosure.

The attachment member 46" may pivotally attach a mounting portion 50" of the medical device mounting assembly 14" to the attachment portion 25" of the base 18". The attachment member 46" may comprise a tab 52" projecting from an arcuate section 54" of the attachment portion 25". The tab 52" may include a pivot hole 56" generally at a front end of the tab and a plurality of locking holes 58" arcuately spaced from one another and extending generally along a back end of the tab adjacent the arcuate section 54" of the attachment portion 25". The locking holes 58" are arranged at an approximately constant distance from the pivot hole 56". The locking holes 58" may be arranged to provide about 45 degrees of pivot adjustment between the mounting portion 50" and the attachment member 46" about a pitch axis extending through the pivot hole 56". In the illustrated embodiment, the pivot hole 56" is disposed at a top of the front end of the tab 52", and a topmost locking hole 58" is disposed near a top of the back end of the tab. It is envisioned that the attachment member 46" may have other shapes and the pivot hole 56" and locking holes 58" may be disposed in other locations.

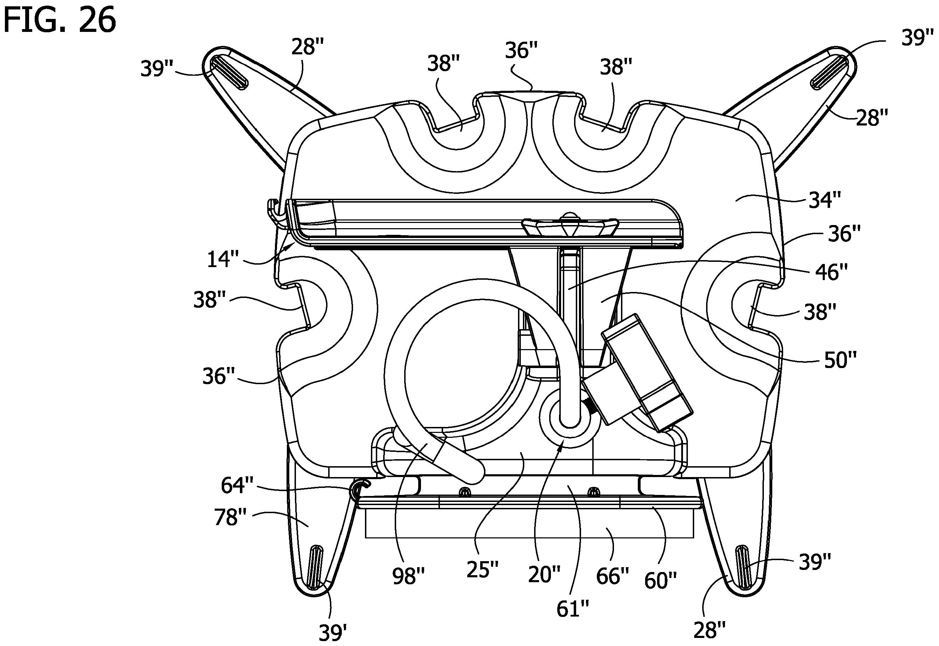

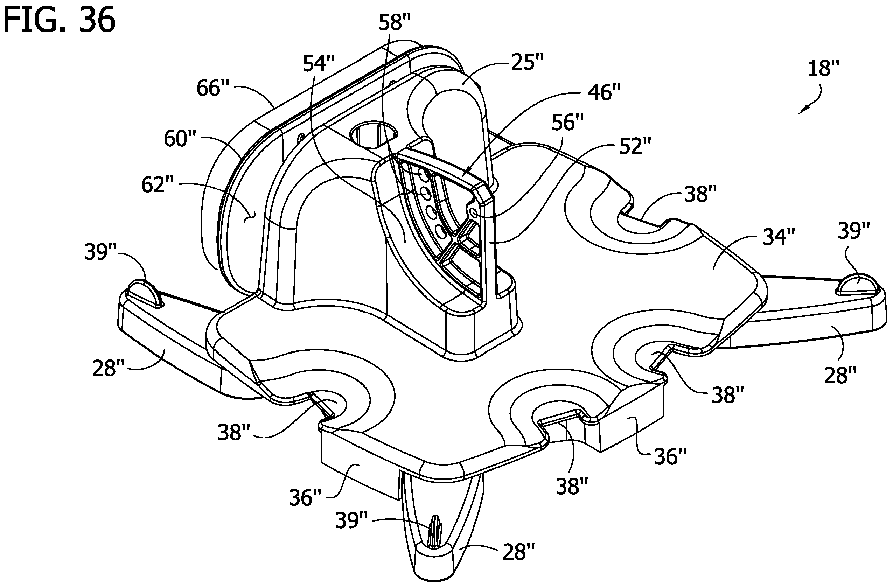

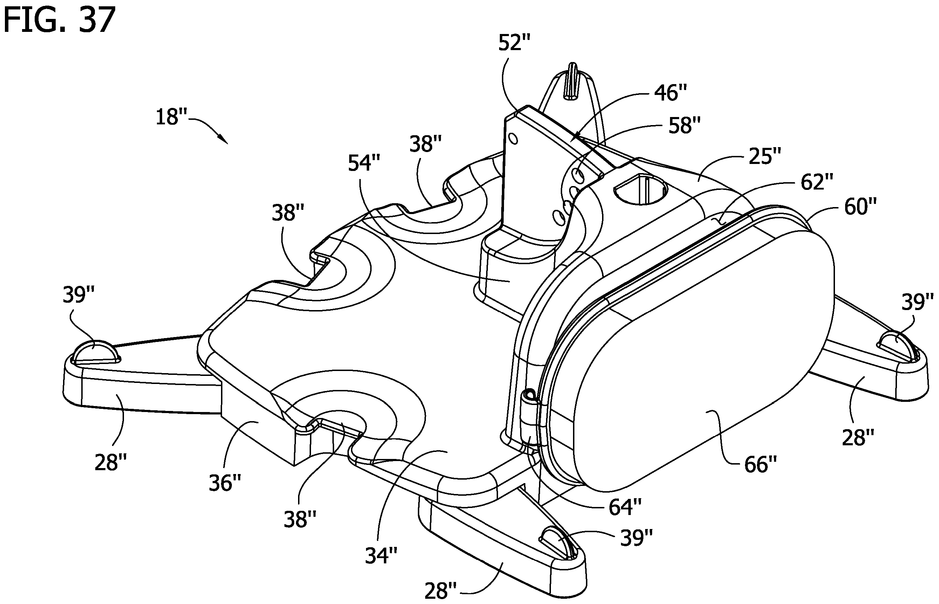

Referring to Figs. 36 and 37, the attachment portion 25" further comprises a spacer 60" mounted at a back of the attachment portion by an extension portion 61" (Fig. 26). An annular gap 62" extending around the extension potion 61" is formed between the flange portion 60" and the arcuate section 54" . The gap 62" may hold any excess medical tubing in a wrapped configuration. A tube holder 64" may be disposed on the flange portion 60" to retain a portion of the medical tubing. The gap 62" and tube holder 64" may individually or collectively be considered a tube holder. A padding 66" may be attached to the spacer 60". The function of the spacer 60" including the padding 66" will be explained below.

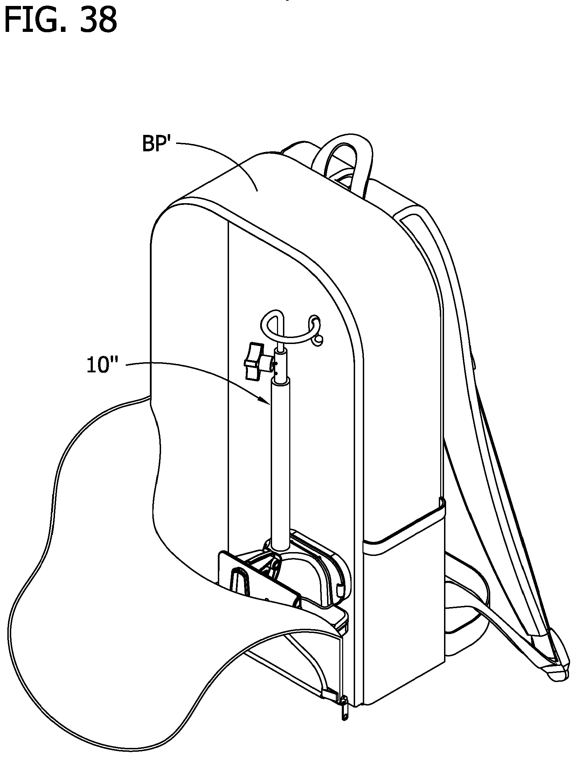

Referring to Fig. 38, the support 10" may be carried in a backpack BP within an internal compartment thereof such that a medical bag 12" is suspended by the support inside the backpack. The feet 28" of the support 10" may be moved to the stowed position (Fig. 33) and the support inserted into the backpack BP. However, the feet 28" may be moved to the deployed position (Fig. 25) to facilitate inserting the support into the backpack BP without departing from the scope of the disclosure. The backpack BP allows the patient the freedom to be mobile while using the support 10" and pump 16" mounted on the support. The same support 10" can be used for table top support out of the backpack BP by moving the feet 28" to the deployed position for stability. The spacer 60", and in particular the padding 66" is positioned to engage the back of the wearer. The contact holds off the base 18" of the support 10", thereby providing space between the wearer's back and the pole 20". Thus, the bag of nutrients will not be likely to be compressed against the wearer's back which could detrimentally affect operation of the feeding system. Additionally, the padding 66" provides a soft surface for engaging the wearer making the assembly more comfortable to wear.

## Claims

1. A medical fluid bag support system (10, 10', 10"), comprising:
a base assembly (18, 18', 18") comprising:
a support portion (24, 24', 24") for supporting the support system on a surface; and,
an attachment portion (25, 25', 25");
a medical device mounting assembly (14, 14', 14") movably attached to the attachment portion, and configured to secure a medical device to the base assembly; and,
a pole assembly (20, 20', 20") comprising a medical bag attachment portion for attaching a medical bag to the pole assembly, the pole assembly being mountable on the base assembly such that when the pole assembly is mounted on the base assembly and the base assembly is on the surface, the medical bag attachment portion is disposed above the base suspending the medical bag above the surface;
wherein the system further comprises at least one foot (28, 28', 28") movably attached to the support portion for movement between a deployed position and a stowed position, wherein the at least one foot is releasably lockable in incremental positions between the stowed position and the deployed position; and, **characterized in that**
either said at least one foot (28, 28") is pivotably attached to the support portion for pivoting between the deployed position and the stowed position, wherein, in the stowed position, each foot is received in a pocket (30, 30") in a bottom of the support portion such that each foot is substantially within an outer perimeter of the support portion; or,
said at least one foot (28') is slidably attached to the support portion for translational movement between the stowed position and the deployed position.

2. The medical fluid bag support system of any one of the preceding claims, wherein each of the at least one foot is independently releasably lockable in incremental positions between the stowed position and the deployed position.

3. The medical fluid bag support system of any one of the preceding claims, wherein the pole assembly comprises a first elongate member and a second elongate member telescopingly received in the first elongate member.

4. The medical fluid bag support system of claim 3, wherein the second elongate member comprises a first straight section for receipt in the first elongate member and a second curved section extending from the first straight section for attaching the medical bag.

5. The medical fluid bag support system of claim 3, further comprising a lock for securing the second elongate member at at least one of a plurality of preselected positions relative to the first elongate member.

6. The medical fluid bag support system of any one of the preceding claims, wherein the support portion and the attachment portion are formed as one piece of material.

7. The medical fluid bag support system of any one of the preceding claims, wherein the medical bag is an IV bag or an enteral feeding bag.

8. The medical fluid bag support system of any one of the preceding claims in combination with a backpack, comprising a backpack portion configured to be supported on a wearer's shoulder and defining an internal storage compartment sized to receive therein the medical fluid bag support system.

9. The combination backpack and medical fluid bag support system of claim 8, wherein the base assembly has a first configuration for supporting the medical bag on the surface and a second configuration for supporting the medical bag in the backpack.

10. The combination backpack and medical fluid bag support system of any one of claims 8 and 9, wherein the internal storage compartment is sized to receive therein the medical fluid support system when the medical fluid bag support system is in the stowed position.

11. The combination backpack and medical fluid bag support system of claim 10 further comprising padding disposed at a back of the base assembly for engaging a wearer of the backpack when the medical fluid bag support system is in the backpack.

## Patentansprüche

1. Tragsystem (10, 10', 10") für medizinische Fluidbeutel, das Folgendes umfasst:
eine Basisbaugruppe (18, 18', 18"), die Folgendes umfasst:
einen Stützabschnitt (24, 24', 24") zum Stützen des Tragsystems auf einer Oberfläche; und
einen Befestigungsabschnitt (25, 25`, 25");
eine Montagebaugruppe (14, 14', 14") für medizinische Vorrichtung, die beweglich an dem Befestigungsabschnitt befestigt und dafür konfiguriert ist, eine medizinische Vorrichtung an der Basisbaugruppe zu befestigen; und
eine Stangenbaugruppe (20, 20', 20"), die einen Befestigungsabschnitt für medizinische Beutel zum Befestigen eines medizinischen Beutels an der Stangenbaugruppe umfasst, wobei die Stangenbaugruppe derart an der Basisbaugruppe montierbar ist, dass, wenn die Stangenbaugruppe an der Basisbaugruppe montiert ist und sich die Basisbaugruppe auf der Oberfläche befindet, der Befestigungsabschnitt für medizinische Beutel oberhalb der Basis angeordnet ist, wobei er den medizinischen Beutel oberhalb der Oberfläche aufhängt;
wobei das System ferner mindestens einen Fuß (28, 28', 28") umfasst, der für eine Bewegung zwischen einer entfalteten Stellung und einer verstauten Stellung beweglich an dem Stützabschnitt befestigt ist, wobei der mindestens eine Fuß lösbar in schrittweisen Stellungen zwischen der verstauten Stellung und der entfalteten Stellung arretierbar ist; und **dadurch gekennzeichnet, dass**
entweder der mindestens eine Fuß (28, 28") zum Schwenken zwischen der entfalteten Stellung und der verstauten Stellung schwenkbar an dem Stützabschnitt befestigt ist, wobei, in der verstauten Stellung, jeder Fuß derart in einer Tasche (30, 30") in einem Unterteil des Stützabschnitts aufgenommen wird, dass sich jeder Fuß im Wesentlichen innerhalb eines Außenumfangs des Stützabschnitts befindet, oder
der mindestens eine Fuß (28') für eine Verschiebungsbewegung zwischen der verstauten Stellung und der entfalteten Stellung verschiebbar an dem Stützabschnitt befestigt ist.

2. Tragsystem für medizinische Fluidbeutel nach einem der vorhergehenden Ansprüche, wobei jeder von dem mindestens einen Fuß unabhängig lösbar in schrittweisen Stellungen zwischen der verstauten Stellung und der entfalteten Stellung arretierbar ist.

3. Tragsystem für medizinische Fluidbeutel nach einem der vorhergehenden Ansprüche, wobei die Stangenbaugruppe ein erstes längliches Element und ein zweites längliches Element, das teleskopartig in dem ersten länglichen Element aufgenommen ist, umfasst.

4. Tragsystem für medizinische Fluidbeutel nach Anspruch 3, wobei das zweite längliche Element einen ersten geraden Bereich zur Aufnahme in dem ersten länglichen Element und einen zweiten gekrümmten Bereich, der sich von dem ersten geraden Bereich aus erstreckt, zum Befestigen des medizinischen Beutels umfasst.

5. Tragsystem für medizinische Fluidbeutel für medizinische Fluidbeutel nach Anspruch 3, das ferner eine Verriegelung zum Sichern des zweiten länglichen Elements in mindestens einer von einer Vielzahl von vorausgewählten Stellungen im Verhältnis zu dem ersten länglichen Element umfasst.

6. Tragsystem für medizinische Fluidbeutel nach einem der vorhergehenden Ansprüche, wobei der Stützabschnitt und der Befestigungsabschnitt als ein Stück Material ausgebildet sind.

7. Tragsystem für medizinische Fluidbeutel nach einem der vorhergehenden Ansprüche, wobei der medizinische Beutel ein Infusionsbeutel oder ein Beutel zur enteralen Ernährung ist.

8. Tragsystem für medizinische Fluidbeutel nach einem der vorhergehenden Ansprüche in Kombination mit einem Rucksack, der einen Rucksackabschnitt umfasst, der dafür konfiguriert ist, auf der Schulter eines Trägers getragen zu werden, und ein inneres Aufbewahrungsfach definiert, das dafür bemessen ist, in demselben das Tragsystem für medizinische Fluidbeutel aufzunehmen.

9. Kombination aus Rucksack und Tragsystem für medizinische Fluidbeutel nach Anspruch 8, wobei die Basisbaugruppe eine erste Konfiguration zum Tragen des medizinischen Beutels auf der Oberfläche und eine zweite Konfiguration zum Tragen des medizinischen Beutels in dem Rucksack aufweist.

10. Kombination aus Rucksack und Tragsystem für medizinische Fluidbeutel nach einem der Ansprüche 8 und 9, wobei das innere Aufbewahrungsfach dafür bemessen ist, in demselben das Tragsystem für medizinisches Fluid aufzunehmen, wenn sich das Tragsystem für medizinische Fluidbeutel in der verstauten Stellung befindet.

11. Kombination aus Rucksack und Tragsystem für medizinische Fluidbeutel nach Anspruch 10, das ferner Polsterung umfasst, die an einer Rückseite der Basisbaugruppe angeordnet ist, zum In-Eingriff-Nehmen eines Trägers des Rucksacks, wenn sich das Tragsystem für medizinische Fluidbeutel in dem Rucksack befindet.

## Revendications

1. Système de support de poche de fluide médicale (10, 10', 10"), comprenant :
un ensemble de base (18, 18', 18") comprenant :
une partie de support (24, 24', 24") pour supporter le système de support sur une surface ; et
une partie de fixation (25, 25', 25") ;
un ensemble de montage de dispositif médical (14, 14', 14") fixé de manière mobile sur la partie de fixation, et configuré pour fixer un dispositif médical sur l'ensemble de base ; et
un ensemble de poteau (20, 20', 20") comprenant une partie de fixation de poche médicale pour fixer une poche médicale sur l'ensemble de poteau, l'ensemble de poteau pouvant être monté sur l'ensemble de base de sorte que lorsque l'ensemble de poteau est monté sur l'ensemble de base et que l'ensemble de base est sur la surface, la partie de fixation de poche médicale est disposée au-dessus de la base, suspendant la poche médicale au-dessus de la surface ;
dans lequel le système comprend en outre au moins un pied (28, 28', 28") fixé de manière mobile sur la partie de support en vue d'un déplacement entre une position déployée et une position rangée, dans lequel l'au moins un pied peut être verrouillé de manière amovible dans des positions incrémentielles entre la position rangée et la position déployée ; et **caractérisé en ce que**
soit ledit au moins un pied (28, 28") est fixé de manière pivotante sur la partie de support pour pivoter entre la position déployée et la position rangée, dans lequel, dans la position rangée, chaque pied est reçu dans une poche (30, 30") dans une partie inférieure de la partie de support, de sorte que chaque pied se situe sensiblement à l'intérieur d'un périmètre extérieur de la partie de support ; soit
ledit au moins un pied (28') est fixé de manière coulissante sur la partie de support en vue d'un mouvement de translation entre la positon rangée et la position déployée.

2. Système de support de poche de fluide médicale selon l'une quelconque des revendications précédentes, dans lequel chacun de l'au moins un pied peut être verrouillé de manière indépendante et libérable dans des positions incrémentielles entre la position rangée et la position déployée.

3. Système de support de poche de fluide médicale selon l'une quelconque des revendications précédentes, dans lequel l'ensemble de poteau comprend un premier élément allongé et un deuxième élément allongé reçu de manière télescopique dans le premier élément allongé.

4. Système de support de poche de fluide médicale selon la revendication 3, dans lequel le deuxième élément allongé comprend une première section droite destinée à être reçue dans le premier élément allongé et une deuxième section incurvée s'étendant depuis la première section droite pour fixer la poche médicale.

5. Système de support de poche de fluide médicale selon la revendication 3, comprenant en outre un verrou pour fixer le deuxième élément allongé au niveau d'au moins une d'une pluralité de positions présélectionnées par rapport au premier élément allongé.

6. Système de support de poche de fluide médicale selon l'une quelconque des revendications précédentes, dans lequel la partie de support et la partie de fixation sont formées en une seule pièce de matériau.

7. Système de support de poche de fluide médicale selon l'une quelconque des revendications précédentes, dans lequel la poche médicale est une poche pour perfusion intraveineuse ou une poche d'alimentation entérale.

8. Système de support de poche de fluide médicale selon l'une quelconque des revendications précédentes en combinaison avec un sac à dos, comprenant une partie de sac à dos configurée pour être supportée sur l'épaule d'un porteur et définissant un compartiment de stockage interne dimensionné pour y recevoir le système de support de poche de fluide médicale.

9. Combinaison de sac à dos et de système de support de poche de fluide médicale selon la revendication 8, dans lequel l'ensemble de base a une première configuration pour supporter la poche médicale sur la surface et une deuxième configuration pour supporter la poche médicale dans le sac à dos.

10. Combinaison de sac à dos et de système de support de poche de fluide médicale selon l'une quelconque des revendications 8 et 9, dans laquelle le compartiment de stockage interne est dimensionné pour y recevoir le système de support de fluide médical lorsque le support de poche de fluide médicale se trouve dans la position rangée.

11. Combinaison de sac à dos et de système de support de poche de fluide médicale selon la revendication 10, comprenant en outre un rembourrage disposé à l'arrière de l'ensemble de base pour engager un porteur du sac à dos lorsque le système de support de poche de fluide médicale se trouve dans le sac à dos.
